# EUROPEAN PATENT APPLICATION

(11) **EP 4 768 074 A2**
(43) Date of publication of application: **01.07.2026**
(21) Application number: 25227203.4
(22) Date of filing: 24.12.2025
(51) Int. Cl.: A61N 5/10

(54) **RADIATION DELIVERY APPARATUS AND COMPUTER-READABLE STORAGE MEDIUM**

(30) Priority: 25.12.2024 CN 202411941636
(71) Applicant: Shanghai United Imaging Healthcare Co., Ltd., Shanghai 201807 (CN)
(72) Inventor: HAN, Wei, Shanghai, 201807 (CN); LIU, Yanfang, Shanghai, 201807 (CN); ZHAO, Fuwei, Shanghai, 201807 (CN); SHA, Kangkang, Shanghai, 201807 (CN); FAN, Houchen, Shanghai, 201807 (CN); ZHANG, Jian, Shanghai, 201807 (CN); YANG, Kun, Shanghai, 201807 (CN); NI, Cheng, Shanghai, 201807 (CN)
(74) Representative: Murgitroyd & Company

(57) **Abstract**

The present disclosure relates to a radiation delivery apparatus (10) and a non-transitory computer-readable storage medium. The radiation delivery apparatus (10) includes a radiation head (11) configured to deliver a radiation beam and rotatable about a rotation axis; and a movement mechanism (12) connected to the radiation head (11). The radiation head (11) is movable along the direction of the rotation axis through the movement mechanism (12) and is movable along the beam direction through the movement mechanism (12).

## Description

### TECHNICAL FIELD

The present disclosure relates to the field of medical device technologies, and in particular, to a radiation delivery apparatus and a non-transitory computer-readable storage medium.

### BACKGROUND

With the continuous advancement of industrial technology, radiation delivery (e.g., radiation therapy, radiation processing, radiation verification) apparatuses have undergone tremendous changes from traditional to intelligent and precise.

In the related art, during the use of a radiation delivery apparatus, it mainly rotates coplanarly around a radiation delivery object to perform radiation delivery operations on a region of interest of the radiation delivery object.

However, the radiation delivery apparatus in the related art has a problem of poor flexibility during a radiation delivery process.

### SUMMARY

A radiation delivery apparatus and a non-transitory computer-readable storage medium are provided in the present disclosure.

In a first aspect, a radiation delivery apparatus is provided in the present disclosure. The radiation delivery apparatus includes:
a radiation head configured to deliver a radiation beam and rotatable about a rotation axis; and
a movement mechanism connected to the radiation head, where the radiation head is movable along a direction of the rotation axis through the movement mechanism, and is movable along a direction of the radiation beam through the movement mechanism.

In some embodiments, the radiation delivery apparatus further includes: a first imaging source, a first imaging detector corresponding to the first imaging source, a second imaging source, and a second imaging detector corresponding to the second imaging source.

In some embodiments, an imaging mode of the first imaging source is different from an imaging mode of the second imaging source.

In some embodiments, the imaging mode of the first imaging source includes a single exposure mode, and the imaging mode of the second imaging source includes a continuous exposure mode.

In some embodiments, an imaging modality of the first imaging source is different from an imaging modality of the second imaging source.

In some embodiments, the imaging modality of the first imaging source includes computed tomography, and the imaging modality of the second imaging source includes digital radiography.

In some embodiments, a type of imaging radiation beam of the first imaging source is different from a type of imaging radiation beam of the second imaging source.

In some embodiments, a type of the first imaging detector is different from a type of the second imaging detector.

In some embodiments, a type of the first imaging detector and a type of the second imaging detector include a flat panel detector.

In some embodiments, a size of the first imaging detector is different from a size of the second imaging detector.

In some embodiments, the movement mechanism includes a first movement channel and a second movement channel, the radiation head is disposed in the first movement channel, and the first movement channel is disposed in the second movement channel, the first movement channel is configured to guide the radiation head to move along a target direction, the second movement channel is configured to guide the radiation head to move along other directions other than the target direction, and the target direction includes the direction of the rotation axis or the beam direction.

In some embodiments, the first movement channel includes a first guide rail, the second movement channel includes a second guide rail, the first guide rail is movably disposed on the second guide rail, the radiation head is movably disposed on the first guide rail, the second guide rail is configured to guide the first guide rail to move along the direction of the rotation axis, and the first guide rail is configured to guide the radiation head to move along the beam direction.

In some embodiments, the movement mechanism includes a swingable frame and a third guide rail, the third guide rail is disposed on the swingable frame, the radiation head is movably disposed on the third guide rail, the swingable frame is configured to drive the third guide rail to swing along the direction of the rotation axis, and the third guide rail is configured to guide the radiation head to move along the beam direction.

In some embodiments, the radiation delivery apparatus further includes a radiation detector disposed on the swingable frame and opposite to the radiation head.

In some embodiments, the radiation head is configured to deliver a radiation beam with a first energy when moving to a first position along the beam direction, and deliver a radiation beam with a second energy different from the first energy when moving to a second position different from the first position along the beam direction.

In some embodiments, the radiation head is configured to deliver the radiation beam focused onto a region of interest on the rotation axis. A distance between the first position and the region of interest is smaller than a distance between the second position and the region of interest. The first energy is smaller than the second energy.

In some embodiments, the radiation head includes a multi-leaf collimator. The multi-leaf collimator includes at least two leaf groups, and the at least two leaf groups are configured to be movable independently relative to each other.

In some embodiments, the radiation head is configured to deliver a radiation beam to a first target site in a region of interest when at least one of the at least two leaf groups moves to a first position along the beam direction, and deliver a radiation beam to a second target site different from the first target site in the region of interest when at least one of the at least two leaf groups moves to a second position different from the first position along the beam direction.

In some embodiments, the radiation head is configured to deliver a radiation beam with a first energy when at least one of the at least two leaf groups moves to a third position along the beam direction, and deliver a radiation beam with a second energy different from the first energy when at least one of the at least two leaf groups moves to a fourth position different from the third position along the beam direction.

In a second aspect, a non-transitory computer-readable storage medium, on which a computer program is stored, is further provided in the present disclosure. When the computer program is executed by a processor, the radiation delivery performed by the radiation delivery apparatus according to any one of the first aspect is implemented.

### BRIEF DESCRIPTION OF THE DRAWINGS

To more clearly illustrate the technical solutions in the embodiments of the present disclosure or the related art, the following briefly introduces the accompanying drawings required for describing the embodiments or the related art. Apparently, the drawings in the following description are only some embodiments of the present disclosure, and for those of ordinary skill in the art, other related drawings can be obtained according to the accompanying drawings without creative efforts.
FIG. 1 is a schematic diagram of a structure of a radiation delivery apparatus in some embodiments of the present disclosure.
FIG. 2 is a schematic diagram of a structure of a radiation delivery apparatus in some embodiments of the present disclosure.
FIG. 3A is a schematic diagram of a structure of a radiation delivery apparatus in some embodiments of the present disclosure.
FIG. 3B is a schematic diagram of a structure of a radiation delivery apparatus in some embodiments of the present disclosure.
FIG. 4 is a schematic diagram of a structure of a radiation head in some embodiments of the present disclosure.
FIG. 5 is a schematic diagram of a radiation delivery system in some embodiments of the present disclosure.
FIG. 6 is a block diagram of a configuration of a control device of a radiation delivery apparatus in some embodiments of the present disclosure.
FIG. 7 is a schematic diagram of an internal configuration of a controller in some embodiments of the present disclosure.

### DETAILED DESCRIPTION OF THE EMBODIMENTS

In order to make the above objectives, features and advantages of the present disclosure more obvious and understandable, specific implementations of the present disclosure are described in detail below with reference to the accompanying drawings. In the following description, many specific details are set forth in order to fully understand the present disclosure. However, the present disclosure can be implemented in many other ways different from those described herein, and those skilled in the art can make similar improvements without departing from the connotation of the present disclosure. Therefore, the present disclosure is not limited by the specific embodiments disclosed below.

In the description of the present disclosure, it should be understood that the orientation or position relationship indicated by the terms "center", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", etc. are based on the orientation or position relationship shown in the accompanying drawings and are merely intended to facilitate the description of the present disclosure and simplify the description, rather than indicating or implying that the indicated device or element must have a particular orientation or be constructed and operated in a particular orientation, and therefore are not to be interpreted as limiting the present disclosure.

The following clearly and completely describes the technical solutions in the embodiments of the present disclosure with reference to the accompanying drawings in the embodiments of the present disclosure. Apparently, the described embodiments are only a part of the embodiments of the present disclosure, rather than all the embodiments. Based on the embodiments in the present disclosure, all other embodiments obtained by those of ordinary skill in the art without creative efforts fall within the protection scope of the present disclosure.

The serial numbers assigned to components herein, such as "first", "second", etc., are only used to distinguish the described objects and do not have any sequential or technical meanings. The terms "connected" and "coupled" mentioned in the present disclosure include direct and indirect connections (couplings) unless otherwise specified. In the description of the present disclosure, it should be understood that the orientations or positional relationships indicated by the terms "upper", "lower", "front", "rear", "left", "right", "vertical", "horizontal", "top", "bottom", "inner", "outer", "clockwise", "counterclockwise", etc. are based on the orientations or positional relationships shown in the accompanying drawings, and are only for the convenience of describing the present disclosure and simplifying the description, rather than indicating or implying that the indicated device or element must have a specific orientation or be constructed and operated in a specific orientation. Therefore, they should not be construed as limiting the present disclosure.

In the present disclosure, unless otherwise clearly specified and limited, a first feature being "on" or "under" a second feature may mean that the first and second features are in direct contact, or the first and second features are in indirect contact through an intermediate medium. Moreover, a first feature being "above", "over" and "on" a second feature may mean that the first feature is directly above or obliquely above the second feature, or simply means that the horizontal height of the first feature is higher than that of the second feature. A first feature being "below", "under" and "beneath" a second feature may mean that the first feature is directly below or obliquely below the second feature, or simply means that the horizontal height of the first feature is lower than that of the second feature.

In the field of radiation delivery (e.g., radiation therapy, radiation processing, radiation verification before radiation therapy, etc.), it is desirable to achieve all-round and multi-angle flexible radiation to meet different radiation needs. In the design process of current radiation delivery apparatuses, an angle of a radiation head is fixed, and the multi-leaf collimator (MLC) in the radiation head is of a single-layer design. During the use of a radiation delivery apparatus, the radiation head with a fixed angle can only rotate coplanarly around a radiation delivery object and cannot perform non-coplanar movement. That is, the current radiation delivery apparatus has poor flexibility during a radiation delivery process. The leaf resolution of a single-layer MLC is usually 5mm or more, making it difficult to achieve higher-resolution dose regulation and a large penumbra. Although technologies such as CyberKnife can achieve a resolution of 2.5mm, they cannot solve the problem of leakage between leaves and cannot protect normal tissues. At the same time, when treating the head and body of a radiation delivery object, it is impossible to meet the needs of different sites.

A radiation delivery apparatus, a system, a control method of the radiation delivery apparatus, a controller, and a non-transitory computer-readable storage medium are provided in the present disclosure, which can improve the flexibility of the radiation delivery apparatus during the radiation delivery process. Of course, the technical solutions provided in the embodiments of the present disclosure are not limited to solving only the above problems, and there are other technical effects, which can be referred to the following embodiment descriptions. Next, the technical solutions of the present disclosure are described in detail.

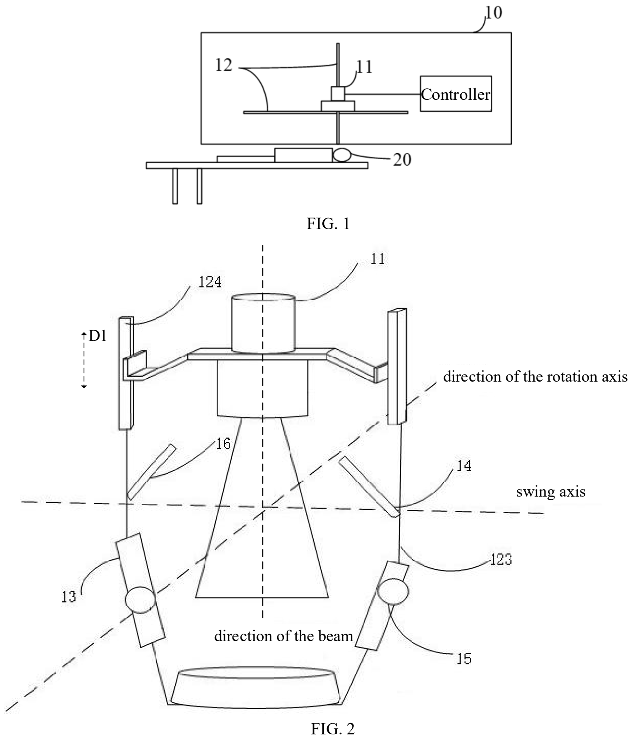

In some embodiments, as shown in FIG. 1, a radiation delivery apparatus 10 is provided. The radiation delivery apparatus 10 includes: a radiation head 11 configured to deliver a radiation beam and rotatable about a rotation axis, and a movement mechanism 12 connected to the radiation head 11. The radiation head 11 is movable along a direction of the rotation axis through the movement mechanism 12, and is movable along a direction of the radiation beam through the movement mechanism 12.

To improve the flexibility of the radiation delivery apparatus 10, the radiation head 11 of the radiation delivery apparatus 10 needs to be able to emit a radiation beam to a region of interest of a radiation delivery object 20 from different angles. The region of interest may be a planned treatment region. To achieve this goal, a corresponding movement mechanism 12 is provided on the basis of a traditional radiation delivery apparatus 10 to assist the radiation head 11 in moving in different directions. A direction of the radiation beam may refer to a straight line direction from a radiation source of the radiation head 11 to an isocenter. A rotation axis of the radiation head 11 may refer to a geometric axis around which the radiation head 11 performs rotational movement, which is configured to adjust a projection direction of a radiation beam to ensure accurate coverage of the region of interest of the radiation delivery object 20 and avoid critical organs. The isocenter may be located on the rotation axis.

The radiation delivery apparatus 10 including the movement mechanism 12 and the radiation head 11 may have a hollow structure, and a support bed for carrying the radiation delivery object 20 may be disposed in the aperture of the hollow structure. A length direction of the support bed may be parallel to a direction of a central axis of an aperture of the hollow structure. Along the length direction of the support bed, the radiation head 11 can emit a radiation beam to different sites of the radiation delivery object 20 on the support bed. Different positions of the radiation head 11 in the beam direction may correspond to different distances between the radiation head 11 and an irradiated region of interest or part of the radiation delivery object 20. It should be noted that, for example, different sites require different radiation delivery distances. For example, in radiation therapy, for the region of interest in the patient's head, the radiation head 11 can be controlled to be close to the region of interest, i.e., the distance is reduced to realize the radiation delivery operation for the region of interest. For the region of interest in the patient's body (e.g., chest, abdomen, etc.), the radiation head 11 can be controlled to be away from the region of interest, i.e., the distance is increased to realize the radiation delivery operation for the region of interest. Thus, by adjusting the position of the radiation head 11 in the beam direction, the radiation needs of different sites can be met.

The movement mechanism 12 may include a movement channel, which may be various types of guide rails, pipes, etc. For example, a docking component of the radiation head 11 may be connected to a clamping groove of a guide rail and move within the clamping groove. For another example, the radiation head 11 may be disposed to move within a pipe. It should be noted that the movement channel needs to meet requirement that the radiation head 11 is movable along the direction of the rotation axis and along the beam direction of the radiation head 11. Therefore, the movement channel may include two channels corresponding to the length direction of the support bed and the beam direction of the radiation head 11, respectively. In this way, by controlling the movement of the radiation head 11 in the two channels, movement along the direction of the rotation axis or along the beam direction of the radiation head 11 is realized.

The radiation head 11 may be configured to emit a radiation beam to the region of interest of the radiation delivery object 20 from different angles. The radiation head 11 may include an accelerating tube, or the radiation head 11 may be connected to an accelerating tube. The radiation head 11 may include devices such as a multi-leaf collimator 18. The accelerating tube is configured to accelerate radiation beam particles or ray particles (e.g., electrons, which are subsequently emitted from the radiation head 11 to form an electron radiation beam) or particles that generate a radiation beam (e.g., electrons, which strike a target to generate photons to form a photon radiation beam). Alternatively, the accelerating tube may also be disposed outside the radiation head 11.

To enable the radiation beam output by the accelerating tube to be suitable for radiation delivery of different sites, the acceleration parameters of the accelerating tube can be controlled by a controller. For example, through the control of acceleration parameters, the radiation head 11 can output at least two energy levels below 7MV, adapting to adverse reactions of the radiation delivery object 20 during the radiation delivery process.

In an entire radiation beam system, the multi-leaf collimator 18 may be disposed downstream of the accelerating tube. Particles accelerated by the accelerating tube or other particles generated by the particles form radiation, which exits the radiation head 11 to form a radiation beam after passing through the multi-leaf collimator 18. Changes of the leaf positions of the multi-leaf collimator 18 can control the shape, size, etc. of the radiation beam, so that a shape of a region covered by the radiation beam output from the radiation head 11 is substantially consistent with a shape of the region of interest of the radiation delivery object 20, ensuring that the radiation operation is only directed at the region of interest, without or reducing the impact on tissues outside the region of interest.

Optionally, the radiation delivery apparatus 10 may further include a controller. The radiation head 11 may be connected to the controller, for example, in communication connection. The radiation head 11 may be disposed in or on the movement channel of the movement mechanism 12.

The controller may be configured to control the radiation head 11 to move along the length direction of the support bed for carrying the radiation delivery object 20 or along the beam direction of the radiation head 11 in the movement channel. The beam direction may refer to a traveling direction of the radiation beam emitted by the radiation head 11, such as a direction perpendicular to a radiation beam exit plane of the radiation head 11 or a direction of a central axis of the radiation beam. As an example, the radiation beam herein may be any one of various types of radiation beams, such as a photon radiation beam, an electron radiation beam, a proton radiation beam, a heavy ion radiation beam, etc. According to different radiation beam types, structures or components involved herein may be adaptively adjusted, but for the sake of brevity, they are not elaborated in the present disclosure.

The controller may be in communication connection with the support bed to control the support bed to move in parallel with the direction of the rotation axis. The radiation delivery object 20 may lie on the support bed along the length direction. When it is necessary to perform a radiation delivery operation on the radiation delivery object 20, the controller may control the support bed to at least partially pass through the aperture of the hollow structure of the radiation delivery apparatus 10 and be fixed at a target position. When the radiation delivery operation for the radiation delivery object 20 is completed, the controller may control the support bed to exit from the target position and move away from the hollow structure.

The controller may be disposed at any position as long as its control function can be realized. The controller may be in communication connection (wired connection or wireless connection) with the radiation head 11 to facilitate controlling the movement of the radiation head 11. The controller may be, but is not limited to, a Pulse Width Modulation (PWM) control chip, a control Integrated Circuit (IC), a voltage reference source, a Phase Locked Loop (PLL) control chip, a current detection chip, a Metal-Oxide-Semiconductor Field-Effect Transistor (MOSFET) driver chip, etc.

The above radiation delivery apparatus 10 includes a radiation head 11 configured to deliver a radiation beam and rotatable about a rotation axis. A movement mechanism 12 is connected to the radiation head 11. The radiation head 11 is movable along the direction of the rotation axis through the movement mechanism 12, and is movable along the beam direction through the movement mechanism 12. By connecting the radiation head 11 to the movement mechanism 12, under the action of the movement of the movement mechanism 12, the radiation head 11 can not only move along the direction of the rotation axis through the movement mechanism 12 but also move along the beam direction through the movement mechanism 12. That is, the radiation delivery apparatus can perform radiation delivery operations from different directions and angles, improving the flexibility of the radiation delivery operation.

To ensure the accuracy of the radiation delivery operation, an imaging source and a corresponding imaging detector may also be provided in the radiation delivery apparatus 10 to image the region of interest during the radiation delivery operation. In some embodiments, as shown in FIG. 2, the radiation delivery apparatus 10 further includes a first imaging source 13, a first imaging detector 14 corresponding to the first imaging source 13, a second imaging source 15, and a second imaging detector 16 corresponding to the second imaging source 15. However, it is not limited thereto, and the radiation delivery apparatus 10 may also include only one imaging source and one corresponding imaging detector. The imaging mode or imaging modality of the first imaging source 13 may be the same as or different from that of the second imaging source 15. The type and/or size of the first imaging detector 14 may be the same as or different from that of the second imaging detector 16.

In the embodiment of the present disclosure, each imaging source and the corresponding imaging detector are disposed opposite to each other on a support structure of the radiation delivery apparatus 10, and the imaging radiation beam generated by each imaging source can pass through the radiation delivery object 20 to be projected onto the corresponding imaging detector.

To ensure the accuracy of imaging, it is necessary to image the region of interest of the radiation delivery object 20 from different angles. Therefore, at least two imaging sources and two imaging detectors may be disposed on the support structure of the radiation delivery apparatus 10, and each imaging source and the corresponding imaging detector are disposed relative to the region of interest, i.e., the imaging radiation beam generated by the imaging source can pass through the region of interest of the radiation delivery object 20 to be transmitted to the imaging detector.

Each imaging source may emit one type of imaging radiation beam or different types of imaging radiation beams. That is, the imaging mode or imaging modality of one of the two imaging sources may be the same as or different from that of the other imaging source. As an example, the first imaging source 13 may be a Computed Tomography (CT) imaging source, and the second imaging source 15 may be a Digital Radiography (DR) imaging source, which have different imaging modalities. As another example, the first imaging source 13 may be a Cone Beam Computed Tomography (CBCT) imaging source, and the second imaging source 15 may be a Fan Beam Computed Tomography (FBCT) imaging source. As yet another example, both the first imaging source 13 and the second imaging source 15 may be Digital Radiography (DR) imaging sources, but the imaging radiation beam energy and/or size of the two imaging sources may be different, i.e., they have different imaging modes. For example, the imaging source may include an X-ray source (e.g., an X-ray tube), a gamma-ray source, etc. The imaging radiation beam may refer to a radiation beam used to image the region of interest of the radiation delivery object 20. Different types of imaging radiation beams may be imaging radiation beams of different energies, imaging radiation beams of different shapes, or imaging radiation beams of different types. Optionally, different types of imaging radiation beams may include a conical imaging radiation beam and a fan-shaped imaging radiation beam. Alternatively, different types of imaging radiation beams may also include a X-ray, a gamma ray, and/or a neutron radiation beam, etc. The embodiment of the present disclosure does not limit the imaging radiation beams.

The two imaging detectors are respectively used to receive the imaging radiation beams generated by the corresponding imaging sources and transmitted through the radiation delivery object 20. For example, the imaging detector may be an amorphous selenium detector, an amorphous silicon detector, a flat panel detector, an arc detector, etc. Each imaging detector may include a plurality of array units or pixels, and its shape may be square, rectangular, arc-shaped, or other shapes. The type and/or size of each imaging detector may be different or the same.

It should be noted that the imaging source and the imaging detector may be fixedly connected to the support structure of the radiation delivery apparatus 10. For example, a fixed connection may be welding, riveting, bonding, bolt connection, hinge connection, etc., which may be detachable connection or non-detachable connection, and may be movable connection or fixed connection.

Further, the first imaging source 13 and the second imaging source 15 are respectively disposed on both sides of the axis where the radiation delivery object 20 is located, or both the first imaging source 13 and the second imaging source 15 are disposed on any side of the axis where the radiation delivery object 20 is located. Disposing the two imaging sources on both sides or the same side of the axis where the radiation delivery object 20 is located ensures that different imaging radiation beams are emitted from different angles, which can more comprehensively image the region of interest of the radiation delivery object 20 during the radiation delivery operation.

Exemplarily, taking the radiation delivery object 20 on the support bed as a dividing line, the first imaging source 13 is fixedly disposed at an upper left position of the axis where the radiation delivery object 20 is located (e.g., the rotation axis around which the radiation head 11 rotates), and the second imaging source 15 may be disposed at a lower left position of the axis where the radiation delivery object 20 is located. At this time, the imaging detector corresponding to the first imaging source 13 is disposed at a lower right position of the axis where the radiation delivery object 20 is located, and the imaging detector corresponding to the second imaging source 15 is disposed at an upper right position of the axis where the radiation delivery object 20 is located.

Alternatively, the first imaging source 13 is fixedly disposed at an upper left position of the axis where the radiation delivery object 20 is located (e.g., the rotation axis around which the radiation head 11 rotates), and the second imaging source 15 may be disposed at an upper right position of the axis where the radiation delivery object 20 is located. At this time, the imaging detector corresponding to the first imaging source 13 is disposed at a lower right position of the axis where the radiation delivery object 20 is located, and the imaging detector corresponding to the second imaging source 15 is disposed at a lower left position of the axis where the radiation delivery object 20 is located.

The above radiation delivery apparatus 10 further includes a first imaging source 13 and a first imaging detector 14 corresponding to the first imaging source 13, and a second imaging source 15 and a second imaging detector 16 corresponding to the second imaging source 15. Moreover, the imaging mode or imaging modality of the first imaging source 13 is different from that of the second imaging source 15. The type and/or size of the first imaging detector 14 is different from that of the second imaging detector 16. The radiation head 11 may be configured to perform radiation delivery based on the first imaging result generated by the first imaging detector and the second imaging result generated by the second imaging detector. By disposing two imaging sources and two imaging detectors opposite to each other on the radiation delivery apparatus 10, the region of interest of the radiation delivery object 20 during the radiation delivery operation can be imaged to guide the correct execution of the radiation delivery process.

In some examples, the radiation delivery apparatus 10 may include a first imaging system and a second imaging system. The first imaging system includes a first imaging source 13 and a first imaging detector 14 corresponding to the first imaging source 13, and the second imaging system includes a second imaging source 15 and a second imaging detector 16 corresponding to the second imaging source 15. Both the first imaging system and the second imaging system may be DR (Digital Radiography) imaging systems. For example, the first imaging source 13 and the second imaging source 15 may each include an X-ray tube. The first imaging source 13 and the second imaging source 15 may be located on the same side or both sides of the radiation head 11. The imaging mode of the first imaging source may include a single exposure mode, the first imaging detector 14 may receive the imaging radiation beam of the X-ray tube of the first imaging source 13 in the single exposure mode, and the first imaging detector 14 may be a flat panel detector or an arc detector matching the first imaging source 13. The imaging mode of the second imaging source may include a continuous exposure mode, the second imaging detector 16 may receive the imaging radiation beam of the X-ray tube of the second imaging source 15 in the continuous exposure mode, and the second imaging detector 16 may be a flat panel detector or a CT detector matching the second imaging source 15. Configuring a dual DR X-ray tube imaging system that includes the first imaging system and the second imaging system, enables motion tracking for the region of interest and optionally further real-time adjustment of the radiation beam which can be performed by the radiation head, significantly improving the real-time accuracy of treatment.

To realize the movement of the radiation head 11 in the length direction of the support bed and the beam direction of the radiation head 11, the movement mechanism 12 may be configured as two movement channels, representing two intersecting different movement trajectories, which may be straight lines or arcs.

In some embodiments, the above movement mechanism 12 includes a first movement channel and a second movement channel, the radiation head 11 is disposed in the first movement channel, the first movement channel may be configured to guide the movement of the radiation head 11, the first movement channel is disposed in the second movement channel, and the second movement channel may be configured to guide the movement of the first movement channel, thereby jointly guiding the movement of the radiation head 11 with the first movement channel. Through two intersecting different movement trajectories, the radiation head 11 can obtain two degrees of freedom of movement at the same time.

In some embodiments, the radiation head 11 is movable along a target direction through the first movement channel, and the radiation head 11 is movable along other directions other than the target direction through the second movement channel. The target direction includes the direction of the rotation axis or the beam direction.

In some specific embodiments, the first movement channel includes a first guide rail 121, and the second movement channel includes a second guide rail 122. The first guide rail 121 is movably disposed on the second guide rail 122. The first guide rail 121 is connected to the radiation head 11.

Specifically, the first guide rail 121 may guide the radiation head 11 to move along the direction of the rotation axis of the radiation head 11, and the second guide rail 122 may guide the first guide rail 121 to move along the beam direction, so as to drive the radiation head 11 on the first guide rail 121 to move along the beam direction (not shown in the figures).

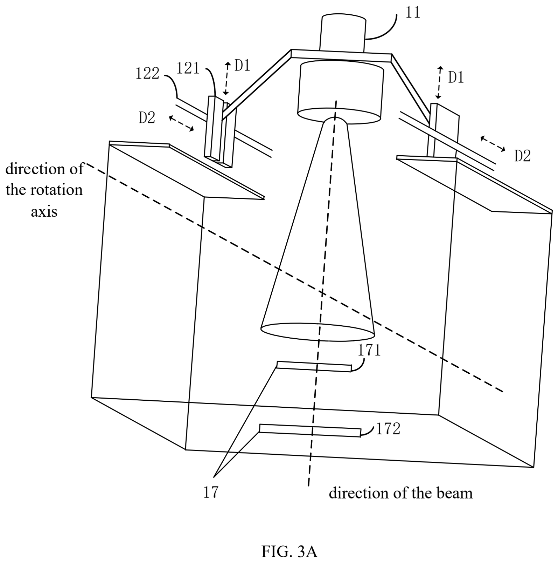

Specifically, as shown in FIG. 3A, the first guide rail 121 may guide the radiation head 11 to move along the beam direction (direction D1 in FIG. 3A). The second guide rail 122 may guide the first guide rail 121 to move along the direction of the rotation axis of the radiation head 11 (direction D2 in FIG. 3A), so as to drive the radiation head 11 on the first guide rail 121 to move along the direction of the rotation axis.

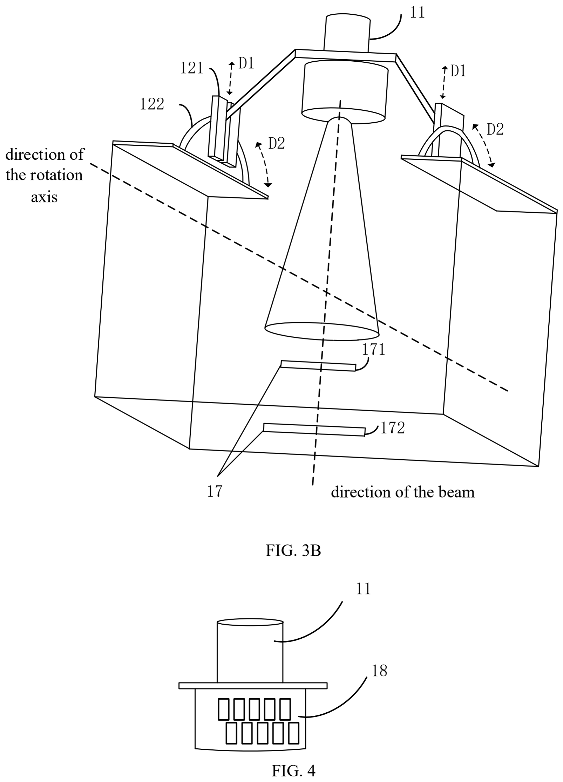

Specifically, as shown in FIG. 3B, the first guide rail 121 may guide the radiation head 11 to move along the beam direction (direction D1 in FIG. 3B). The second guide rail 122 may guide the first guide rail 121 to move along other directions other than the target direction (e.g., direction D2 in FIG. 3B, which is an arc trajectory direction), so as to drive the radiation head 11 on the first guide rail 121 to move along the above other directions.

The embodiment of the present disclosure does not specifically limit other directions. Other directions are predetermined specific trajectory directions perpendicular to or complementary to the target direction, rather than irregular arbitrary directions. Through the division of labor and cooperation of the two movement channels, the radiation head 11 can have multi-dimensional movement capabilities to meet the radiation delivery needs of different sites and distances.

In the embodiment of the present disclosure, the first movement channel and the second movement channel may correspond to the length direction of the support bed and the beam direction of the radiation head 11, respectively. Assuming that the first movement channel is the length direction of the support bed, the first movement channel may be disposed parallel to the length of the support bed, and the second movement channel may be disposed parallel to the beam direction of the radiation head 11. The first movement channel and the second movement channel may be mutually perpendicular channels. For example, the first movement channel is a horizontal channel disposed parallel to the length of the support bed, and the second movement channel is a vertical channel disposed along the height of the support bed. Alternatively, the second movement channel may be configured as an arc channel, and the first movement channel is configured as a vertical channel disposed perpendicular to a tangent of the arc channel. It should be noted that the first movement channel is not limited to the specific implementation of the first guide rail 121, and the second movement channel is not limited to the specific implementation of the second guide rail 122. Other adaptive transmission and guiding structures may also be adopted according to the movement accuracy, load capacity, and spatial layout requirements of the apparatus, as long as the movement trajectories guided by the first movement channel and the second movement channel intersect.

In some embodiments, the radiation head 11 is disposed in the first movement channel, and the first movement channel is disposed in the second movement channel. When the controller controls the radiation head 11 to move in the first movement channel, the second movement channel may remain relatively fixed with the first movement channel. When the controller controls the first movement channel to move in the second movement channel, the first movement channel may remain relatively fixed with the radiation head 11, i.e., the first movement channel and the radiation head 11 may move synchronously in the second movement channel.

Exemplarily, assuming that the first movement channel is parallel to the length direction of the support bed, and the second movement channel is parallel to the beam direction of the radiation head 11, then the second movement channel is disposed outside the first movement channel, and the first movement channel may be disposed within the second movement channel via rollers that slide up and down. When the radiation head 11 moves along the beam direction, the first movement channel and the radiation head 11 can slide up and down synchronously via the rollers, realizing that mechanism 12 guides the radiation head 11 to move in the beam direction.

Assuming that the first movement channel is parallel to the beam direction of the radiation head 11, and the second movement channel is parallel to the length direction of the support bed, similarly, the second movement channel is disposed outside the first movement channel, and the second movement channel is nested on the hollow structure of the first movement channel. When the radiation head 11 moves along the length direction of the support bed, the first movement channel and the radiation head 11 can slide synchronously on the second movement channel, realizing that the movement mechanism 12 guides the radiation head 11 to move in the length direction of the support bed.

It should be noted that the moving lengths of the first movement channel and the second movement channel may be determined based on a length value of the support bed and a predetermined radiation beam moving distance. The controller can control the radiation head 11 to move in both directions at the same time, or control the radiation head 11 to move in any one of the directions.

To ensure the normal operation of the radiation head 11 of the radiation delivery apparatus 10 on the movement channel, a support structure may also be provided in the radiation delivery apparatus 10, and the second movement channel is fixedly connected to the support structure of the radiation delivery apparatus 10. The first movement channel, the second movement channel, and the radiation head 11 are supported by the support structure.

The above movement mechanism 12 includes a first movement channel and a second movement channel, the radiation head 11 is disposed in the first movement channel, and the first movement channel is disposed in the second movement channel. The radiation head 11 is movable along a target direction through the first movement channel, and the radiation head 11 is movable along other directions other than the target direction through the second movement channel. The target direction includes the direction of the rotation axis or the beam direction. The first movement channel includes a first guide rail 121, the second movement channel includes a second guide rail 122, the first guide rail 121 is movably disposed on the second guide rail 122, the radiation head 11 is movably disposed on the first guide rail 121, the second guide rail 122 is configured to guide the first guide rail 121 to move along the direction of the rotation axis, so as to drive the radiation head 11 on the first guide rail 121 to move along the direction of the rotation axis, and the first guide rail 121 is configured to guide the radiation head 11 to move along the beam direction. By respectively setting two different movement channels in the direction of the rotation axis or the beam direction, and the radiation head 11, the first movement channel, and the second movement channel are gradually nested, the movement of the radiation head 11 in the two directions can be accurately controlled, increasing the degree of freedom of the radiation head 11 during the radiation operation and improving the flexibility of radiation therapy.

The radiation head 11 can not only move along the length direction of the support bed or the beam direction of the radiation head 11 but also rotate coplanarly around the support bed. In some embodiments, referring to FIG. 2, the movement mechanism 12 includes a swingable frame 123 and a third guide rail 124, the third guide rail 124 is disposed on the swingable frame 123, the radiation head 11 is movably disposed on the third guide rail, the swingable frame 123 is configured to drive the third guide rail 124 to swing along the direction of the rotation axis, so as to drive the radiation head 11 on the third guide rail 124 to swing along the direction of the rotation axis, and the third guide rail 124 is configured to guide the radiation head 11 to move along the beam direction.

It should be noted that the third guide rail 124 has the same function as the first guide rail 121, both being configured to guide the radiation head 11 to move along the beam direction (direction D 1 in FIG. 2). Referring to FIG. 2, a swing axis may be an axis around which the swingable frame 123 swings. For example, the swingable frame 123 is connected to an external structure through two opposite rotating bearings (not shown) on the left and right sides and realizes swinging or rotating through the two rotating bearings. At this time, the rotation axis may be in a straight line direction formed by connecting t central axes of the two rotating bearings. The swing axis may be located in a tangential direction of the radiation delivery apparatus 10, and the rotation axis may be an axis around which the radiation head 11 rotates when rotating around the radiation delivery object 20, such as the length direction of the support bed. In some examples, the swingable frame 123 may rotate or swing 60 degrees around the swing axis. In some examples, the swingable frame 123 may rotate or swing 360 degrees or nearly 360 degrees around the swing axis.

In the embodiment of the present disclosure, the radiation head 11 may be disposed on the third guide rail 124, and the third guide rail 124 is disposed on the swingable frame 123. The swingable frame 123 can drive the radiation head 11 to rotate coplanarly around the radiation delivery object 20. The coplanar rotation mentioned here essentially refers to rotating clockwise or counterclockwise around the length direction of the radiation delivery object 20 or the support bed (e.g., a head-foot direction of a patient). The controller can control the rotation of the swingable frame 123 according to the radiation delivery needs, thereby driving the rotation of the radiation head 11.

The swingable frame 123 may be disposed inside the support structure, and the imaging sources (the first imaging source 13 and the second imaging source 15) and the imaging detectors (the first imaging detector 14 and the second imaging detector 16) may all be disposed on the support structure. In this way, the swingable frame 123 may affect the imaging radiation beams output by the imaging sources (the first imaging source 13 and the second imaging source 15). Therefore, in some embodiments, an overlapping part of the swingable frame 123 is hollowed out. In this way, the imaging radiation beam generated by each imaging source (the first imaging source 13 and the second imaging source 15) can be emitted to the position of the region of interest of the radiation delivery object 20 through the hollowed-out position to image the region of interest. By setting the overlapping part of the swingable frame 123 that may overlap with the imaging radiation beams generated by the imaging sources (the first imaging source 13 and the second imaging source 15) as a hollow structure, the imaging radiation beams can reach the radiation delivery object 20 through the hollow structure, avoiding the impact of the swingable frame 123 on the imaging process.

In some embodiments, when the radiation head 11 moves to a first position along the beam direction, the radiation head 11 delivers a radiation beam with a first energy. When the radiation head 11 moves to a second position different from the first position along the beam direction, the radiation head 11 delivers a radiation beam with a second energy different from the first energy. The first position and the second position may represent different radiation distances (treatment distances) to cover regions of interest of different volumes. Thus, it is possible to control radiation beams of different energies to perform radiation on the radiation delivery object 20.

At the same time, the radiation head 11 can approach or move away from the region of interest along the third guide rail 124. For example, by controlling the radiation head 11 to be at different positions on the third guide rail 124, the position of the radiation head 11 in the beam direction is different, thereby making the distance between the radiation head 11 and the region of interest different, and the radiation beam energy delivered by the radiation head 11 to the region of interest is also different. In general, the closer the distance between the radiation head 11 and the region of interest, the lower the radiation beam energy delivered to the region of interest. The farther the distance between the radiation head 11 and the region of interest, the higher the radiation beam energy delivered to the region of interest. That is, if the distance between the first position and the region of interest is smaller than the distance between the second position and the region of interest, the first energy is smaller than the second energy. The two energy levels are respectively adapted to different SADs (Source to Axis Distance, which may refer to the distance from the radiation source to the center of a planned treatment region). The first energy may be adapted to fine irradiation of small-volume regions of interest, and the second energy may be adapted to uniform coverage of large-volume regions of interest.

The above movement mechanism 12 includes a swingable frame 123 and a third guide rail 124. The third guide rail 124 is disposed on the swingable frame 123, and the radiation head 11 is movably disposed on the third guide rail. The swingable frame 123 is configured to drive the third guide rail 124 to swing around the swing axis direction, so as to drive the radiation head 11 on the third guide rail 124 to swing along the direction of the rotation axis. Thus, the radiation head 11 can move in the direction of the rotation axis. For example, the radiation head 11 can move in an arc or circular trajectory on a plane where the rotation axis is located and perpendicular to the swing axis. The third guide rail 124 is configured to guide the radiation head 11 to move along the beam direction. By disposing the swingable frame 123 in the radiation delivery apparatus 10, the rotation of the swingable frame 123 is controlled to drive the radiation head 11 to move or swing along the direction of the rotation axis, increasing the movement freedom of the radiation head 11.

In some embodiments, referring to FIG. 3A and FIG. 3B, the radiation delivery apparatus 10 further includes a radiation detector 17, which is disposed on the swingable frame 123 and opposite to the radiation head 11.

The radiation detector 17 may refer to a dose detection device used to detect the dose passing through the region of interest. Optionally, the radiation detector 17 can realize at least one of the following movement modes: moving along the beam direction, rotating around the beam direction, and moving on a plane perpendicular to the beam direction. As an example, the radiation detector 17 may be disposed on a guide rail similar to the third guide rail 124, on the opposite side of the radiation head 11, and can adjust its position in coordination with the movement of the radiation head 11. For example, when the radiation head 11 moves closer to the target object, the radiation detector 17 also moves closer to the radiation delivery object 20, and when the radiation head 11 moves away from the radiation delivery object 20, the radiation detector 17 also moves away from the radiation delivery object 20. However, it is not limited thereto, and the radiation detector 17 may also be fixedly disposed on the swingable frame 123.

The radiation detector 17 may include an electronic portal imaging device 171. The electronic portal imaging device 171 may be disposed on the swingable frame 123 and opposite to the radiation head 11. For example, the electronic portal imaging device 171 is disposed on the other side of the radiation head 11 to ensure that the radiation beam output by the radiation head 11 strikes the electronic portal imaging device 171 after passing through the region of interest. By disposing the radiation detector 17 on the radiation delivery apparatus 10, the dose penetrating the region of interest can be detected, so that the absorbed dose of the region of interest can be accurately estimated based on the detected dose. At the same time, the dose passing through the region of interest can also be shielded to prevent the dose from leaking out of the radiation delivery apparatus 10 and avoid affecting normal tissues. However, it is not limited thereto, and alternatively or additionally, the radiation detector 17 may also include other types of detectors such as a dosimeter.

In addition, a radiation beam shield 172 may be disposed below the electronic portal imaging device 171, and the radiation beam shield 172 is configured to ensure that the main part (or a main radiation beam) of the radiation beam does not leak out of the radiation delivery apparatus 10. Both the electronic portal imaging device 171 and the radiation beam shield 172 are disposed on the swingable frame 123 to ensure synchronous rotation with the radiation head 11.

The electronic portal imaging device 171 and the radiation beam shield 172 may be integrally arranged or separately arranged. Regardless of the arrangement, both the electronic portal imaging device 171 and the radiation beam shield 172 are disposed on the other side of the radiation head 11.

When the radiation head 11 is disposed directly above the support bed, both the electronic portal imaging device 171 and the radiation beam shield 172 are disposed below the support bed. After the radiation head 11 emits a radiation beam, the radiation beam passes through the region of interest of the radiation delivery object 20, the dose strikes the electronic portal imaging device 171, and part of the dose strikes the radiation beam shield 172 through the edge of the electronic portal imaging device 171 or through the electronic portal imaging device 171. The dose of the region of interest of the radiation delivery object 20 can be predicted based on the dose or image determined by the electronic portal imaging device 171. That is, the radiation head 11, the electronic portal imaging device 171, and the radiation beam shield 172 remain on the same straight line.

It can be understood that the area of the radiation beam shield 172 may be larger than that of the electronic portal imaging device 171 to ensure that the main part (or the main radiation beam) of the radiation beam does not leak out of the radiation delivery apparatus 10.

It should be emphasized that both the electronic portal imaging device 171 and the radiation beam shield 172 are perpendicular to the radiation beam, i.e., the radiation head 11 is perpendicular to the electronic portal imaging device 171 and the radiation beam shield 172 respectively. The electronic portal imaging device 171 and the radiation beam shield 172 can ensure the perpendicular relationship with the radiation head 11 through their respective transmission mechanisms according to their respective arc tracks. Alternatively, the radiation head 11, the electronic portal imaging device 171, and the radiation beam shield 172 may be configured as a whole, and the swing angle may be adjusted through a shaft, so that the radiation head 11 remains perpendicular to the electronic portal imaging device 171 and the radiation beam shield 172 respectively. By defining the arrangement relationship between the radiation head 11, the electronic portal imaging device 171, and the radiation beam shield 172, the dose reception of the radiation delivery object 20 during the radiation delivery process can be determined, and the main part of the radiation beam can be prevented from leaking out of the radiation delivery apparatus 10, reducing a thickness of a shielding room and saving a hospital construction cost.

Next, a multi-leaf collimator 18 inside the radiation head 11 is described in detail. In some embodiments, as shown in FIG. 4, the radiation head 11 includes a multi-leaf collimator, the multi-leaf collimator includes at least two leaf groups, and the at least two leaf groups are configured to be movable independently relative to each other. The multi-leaf collimator may be a multi-layer multi-leaf collimator 18, and at least two leaf groups in the multi-layer multi-leaf collimator 18 can move independently relative to each other. The at least two leaf groups are arranged along the beam direction. While the radiation head 11 moves, the at least two leaf groups can move.

One or more leaf groups are movable along the beam direction. At least two leaf groups in the multi-layer multi-leaf collimator 18 can move independently relative to each other, but it is not limited thereto, and one or more leaf groups can also realize other movements. For example, the radiation head 11 includes a multi-layer multi-leaf collimator 18, and at least two leaf groups in the multi-layer multi-leaf collimator 18 can move independently relative to each other.

The leaf groups may be configured to adjust the direction, shape, etc. of the radiation beam output from the radiation head 11, and the adjusted radiation beam can be configured to perform radiation operations on the radiation delivery object 20. Each leaf group may include a plurality of leaves. Using the plurality of leaves of the leaf groups, parameters such as the shape of the radiation beam, the resolution of the radiation field, and the dose rate can be controlled.

The radiation head 11 may include a multi-layer multi-leaf collimator 18. As an example, there may be at least two leaf groups disposed at different horizontal heights. That is, the heights of different leaf groups are different.

Taking the number of leaf groups as two as an example, in some embodiments, referring to FIG. 4, the at least two leaf groups include a first leaf group and a second leaf group, and the first leaf group and the second leaf group are arranged in a staggered configuration.

The leaf group includes a plurality of narrow leaves. Optionally, the leaves in the leaf group are set as independently movable leaves, or each leaf can move together as a leaf group. When the leaf groups are arranged in a staggered configuration, the leaf group farther from the accelerating tube can shield the radiation passing through the gaps between the leaves of the leaf group closer to the radiation source. Optionally, at least two leaf groups in the multi-leaf collimator 18 may always maintain a staggered arrangement. Optionally, the multi-leaf collimator 18 can adjust the state of the leaf groups in the following two scenarios: when a radiation beam limiting device performs a radiation beam limiting function, or when the multi-leaf collimator 18 runs to a specified position. In any of the above scenarios, the multi-leaf collimator 18 can drive at least two leaf groups inside it to move relative to each other, so that the leaf groups maintain or switch to a staggered arrangement.

In some embodiments, when at least one of the at least two leaf groups moves to a first position along the beam direction, the radiation head 11 delivers a radiation beam to a first target site.

When at least one of the at least two leaf groups moves to a second position different from the first position along the beam direction, the radiation head 11 delivers a radiation beam to a second target site different from the first target site.

When at least one of the at least two leaf groups moves to a third position along the beam direction, the radiation head 11 delivers a radiation beam with a first energy.

When at least one of the at least two leaf groups moves to a fourth position different from the third position along the beam direction, the radiation head 11 delivers a radiation beam with a second energy different from the first energy.

Specifically, the multi-leaf collimator 18 can translate integrally along the beam direction, so that the multi-leaf collimator 18 approaches or moves away from the radiation delivery object 20. Alternatively, the multi-leaf collimator 18 can also rotate along the beam direction while translating along the beam direction. Taking an initial state of the multi-leaf collimator 18 being perpendicular to the beam direction as an example, if the multi-leaf collimator 18 rotates along the beam direction, an angle between the multi-leaf collimator 18 and the beam direction changes accordingly.

The movement modes of at least two leaf groups within the multi-leaf collimator 18 may be different or the same. For example, they can move synchronously along the beam direction, move sequentially, or only part of the leaf groups move. The movement process of the leaf groups may be continuous movement or stepwise movement. The movement mode of the leaf groups can be set by the user or obtained according to the radiation delivery needs.

When the multi-leaf collimator 18 as a whole approaches the radiation delivery object 20, the resolution of the radiation field obtained by the radiation beam output through the multi-leaf collimator 18 is improved, the penumbra of the radiation field is reduced, and the range of the radiation field is reduced. On the contrary, when the resolution of the radiation field obtained by the radiation beam output through the multi-leaf collimator 18 is reduced, the penumbra is increased, and the range is increased. When the multi-leaf collimator 18 rotates, the shape of the radiation field obtained by the radiation output through the multi-leaf collimator 18 changes correspondingly. By setting the multi-leaf collimator 18 that is movable along the beam direction, the effect of controlling the radiation and changing the parameters of the radiation field can be achieved to meet different radiation delivery needs.

The above radiation head 11 includes a multi-layer multi-leaf collimator 18, and at least two leaf groups in the multi-layer multi-leaf collimator 18 can move independently relative to each other. The at least two leaf groups are arranged along the beam direction. The at least two leaf groups include a first leaf group and a second leaf group, and the first leaf group and the second leaf group are arranged in a staggered configuration. While the radiation head 11 moves, the at least two leaf groups can move. When at least one of the at least two leaf groups is located at a first position in the beam direction, the radiation head 11 delivers a radiation beam to a first target site. When at least one of the at least two leaf groups is located at a second position different from the first position in the beam direction, the radiation head 11 delivers a radiation beam to a second target site different from the first target site. When at least one of the at least two leaf groups is located at a third position in the beam direction, the radiation head 11 delivers a radiation beam with a first energy. When at least one of the at least two leaf groups is located at a fourth position different from the third position in the beam direction, the radiation head 11 delivers a radiation beam with a second energy different from the first energy. By setting at least two leaf groups and arranging the two leaf groups in a staggered configuration in the height direction, the leakage of the radiation beam can be reduced, the impact of the leaked radiation beam on normal tissues can be avoided, and the safety of the radiation delivery process can be improved. In addition, since the relative position between at least two leaf groups within the multi-leaf collimator 18 and the radiation delivery object 20 remains unchanged during the overall movement of the radiation head 11, the two leaf groups can maintain a good cooperative state.

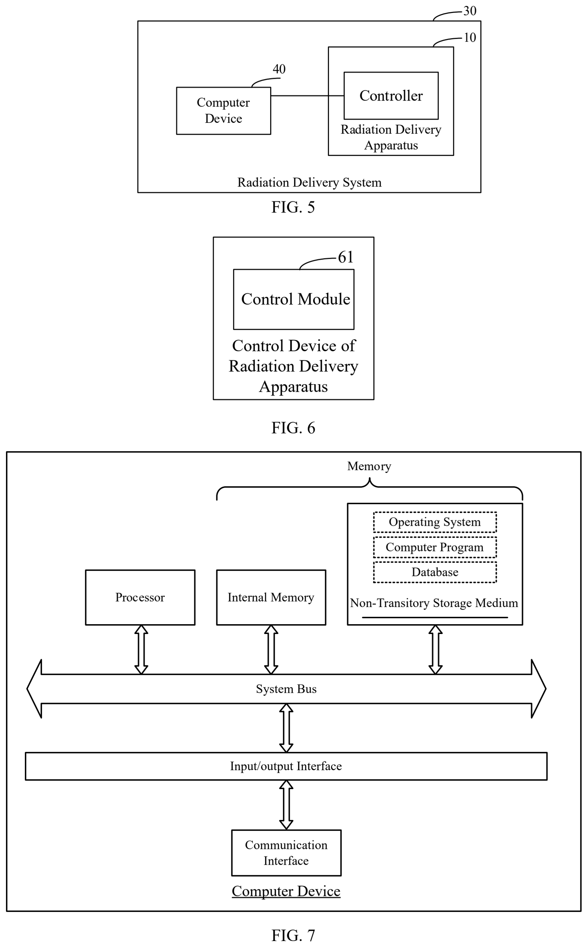

In some embodiments, as shown in FIG. 5, a radiation delivery system 30 is provided. The radiation delivery system 30 includes a radiation delivery apparatus 10 and a computer device 40. The computer device 40 is configured to receive an instruction triggered by a user and send it to a controller in the radiation delivery apparatus 10. The controller is configured to control the radiation head 11 to move along the direction of the rotation axis or the beam direction of the radiation head in the movement mechanism 12 according to the instruction.

In the embodiment of the present disclosure, since the radiation delivery apparatus 10 has a certain radiation to the human body during the radiation delivery process, the radiation delivery apparatus 10 is in a separate operation space during operation. Through the connection between the computer device 40 outside the operation space and the radiation delivery apparatus 10, the user can trigger an operation instruction on the computer device 40, and the operation instruction can be transmitted to the controller of the radiation delivery apparatus 10. The controller can control the radiation head 11 to move along the direction of the rotation axis or the beam direction in the movement mechanism 12 based on the instruction.

The above radiation delivery system 30 includes a radiation delivery apparatus 10 and a computer device 40. The computer device 40 is configured to receive an instruction triggered by a user and send it to a controller in the radiation delivery apparatus 10. The controller is configured to control the radiation head 11 to move along the direction of the rotation axis or the beam direction of the radiation head in the movement mechanism 12 according to the instruction. The radiation delivery apparatus 10 in the radiation delivery system 30 is provided with a movement mechanism 12, and the radiation head 11 is disposed in the movement mechanism 12. The user triggers an instruction through the computer device 40, and after receiving the instruction, the controller controls the movement of the radiation delivery apparatus 10 in the movement mechanism 12, so that the radiation delivery apparatus 10 can move in both the direction of the rotation axis and the beam direction of the radiation head. That is, the radiation delivery apparatus 10 can realize radiation delivery operations from different directions and angles, improving the flexibility of the radiation delivery operation.

Taking the radiation delivery apparatus as a radiation delivery device as an example, in an exemplary embodiment, a control method of the radiation delivery apparatus is provided. Taking the method applied to a controller as an example, it includes the following implementation.

In the embodiment of the present disclosure, the user can trigger an instruction through a computer device connected to the controller, and the computer device forwards the instruction to the controller. When the controller receives the instruction, it controls the radiation head to move along the direction of the rotation axis or the beam direction of the radiation head in the movement mechanism.

In the above control method of the radiation delivery apparatus, in response to an instruction triggered by a user, the radiation head is controlled to move along the direction of the rotation axis or the beam direction of the radiation head in the movement channel. By controlling the movement of the radiation delivery apparatus in the movement mechanism, the controller can realize the movement of the radiation delivery apparatus in both the direction of the rotation axis and the beam direction of the radiation head. That is, the radiation delivery apparatus can realize radiation delivery operations from different directions and angles, improving the flexibility of the radiation delivery operation.

It should be understood that although the various steps in the flowcharts involved in the above embodiments are shown in sequence according to the arrows, these steps are not necessarily executed in sequence according to the arrow instructions. Unless explicitly stated herein, the execution of these steps is not strictly limited in order, and these steps can be executed in other orders. Moreover, at least part of the steps in the flowcharts involved in the above embodiments may include multiple steps or multiple stages. These steps or stages are not necessarily executed at the same time, but can be executed at different times. The execution order of these steps or stages is not necessarily sequential, but can be rotated or alternately executed with at least part of the steps or stages in other steps or other steps.

Based on the same inventive concept, a control device of the radiation delivery apparatus for implementing the above-mentioned control method of the radiation delivery apparatus is further provided in the embodiments of the present disclosure. The implementation scheme for solving the problem provided by the device is similar to the implementation scheme recorded in the above method, so the specific limitations in one or more embodiments of the control device of the radiation delivery apparatus provided below can refer to the limitations on the control method of the radiation delivery apparatus above, and will not be repeated here.

In an exemplary embodiment, as shown in FIG. 6, a control device of a radiation delivery apparatus is provided, including a control module 61.

The control module 61 is configured to control the radiation head to move along the direction of the rotation axis or the beam direction of the radiation head in the movement mechanism in response to an instruction triggered by a user.

Each module in the above control device of the radiation delivery apparatus can be fully or partially implemented by software, hardware, and combinations thereof. The above modules can be embedded in or independent of the processor in the computer device in the form of hardware, or stored in the memory in the computer device in the form of software, so that the processor can call and execute the operations corresponding to the above modules.

In an exemplary embodiment, a controller is provided. The controller may be a server, and its internal structure diagram may be as shown in FIG. 7. The controller includes a processor, a memory, an input/output interface (I/O), and a communication interface. The processor, the memory, and the input/output interface are connected through a system bus, and the communication interface is connected to the system bus through the input/output interface. The processor of the controller is configured to provide computing and control capabilities. The memory of the controller includes a non-transitory storage medium and an internal memory. The non-transitory storage medium stores an operating system, a computer program, and a database. The internal memory provides an environment for the operation of the operating system and the computer program in the non-transitory storage medium. The database of the controller is configured to store data for controlling the radiation delivery apparatus to perform radiation delivery on the target object. The input/output interface of the controller is configured to exchange information between the processor and external devices. The communication interface of the computer device is configured to communicate with an external terminal through a network connection. The computer program is executed by the processor to implement a control method of the radiation delivery apparatus.

Those skilled in the art can understand that the structure shown in FIG. 7 is only a block diagram of a part of the structure related to the solution of the present disclosure, and does not constitute a limitation on the computer device to which the solution of the present disclosure is applied. The specific computer device may include more or fewer components than those shown in the figure, or combine some components, or have different component arrangements.

In an exemplary embodiment, a controller is provided, including a memory and a processor. A computer program is stored in the memory, and the processor controls the radiation delivery apparatus to perform radiation delivery on a target object when executing the computer program.

In an embodiment, a non-transitory computer-readable storage medium is provided, on which a computer program is stored, and when the computer program is executed by a processor, the radiation delivery apparatus is controlled to perform radiation delivery on a target object.

In an embodiment, a computer program product is provided, including a computer program, which controls the radiation delivery apparatus to perform radiation delivery on a target object when executed by a processor.

It should be noted that the user information (including but not limited to user device information, user personal information, etc.) and data (including but not limited to data used for analysis, stored data, displayed data, etc.) involved in the present disclosure are all information and data authorized by the user or fully authorized by all parties. The collection, use, and processing of relevant data need to comply with relevant regulations.

Those of ordinary skill in the art can understand that all or part of the processes in the methods of the above embodiments can be implemented by instructing relevant hardware through a computer program. The computer program can be stored in a non-transitory computer-readable storage medium. When the computer program is executed, it can include the processes of the embodiments of the above methods. Any reference to a memory, database, or other medium used in the various embodiments provided in the present disclosure may include at least one of a non-transitory memory and a volatile memory. The non-transitory memory may include a read-only memory (ROM), a magnetic tape, a floppy disk, a flash memory, an optical memory, a high-density embedded non-transitory memory, a resistive random access memory (ReRAM), a magnetoresistive random access memory (MRAM), a ferroelectric random access memory (FRAM), a phase change memory (PCM), a graphene memory, etc. The volatile memory may include a random access memory (RAM) or an external cache memory, etc. As an illustration and not a limitation, the RAM may be in various forms, such as a static random access memory (SRAM) or a dynamic random access memory (DRAM). The databases involved in the various embodiments provided in the present disclosure may include at least one of a relational database and a non-relational database. The non-relational database may include a distributed database based on a blockchain, etc., but is not limited thereto. The processors involved in the various embodiments provided in the present disclosure may be general-purpose processors, central processing units, graphics processors, digital signal processors, programmable logic devices, data processing logic devices based on quantum computing, artificial intelligence (AI) processors, etc., but are not limited thereto.

Without violating physical principles, the technical features of the above embodiments can be arbitrarily combined. To make the description concise, not all possible combinations of the technical features in the above embodiments are described. However, as long as there is no contradiction in the combination of these technical features, they should be considered as the scope of the present disclosure.

The above embodiments only express several implementation modes of the present disclosure, and their descriptions are relatively specific and detailed, but they should not be construed as limiting the patent scope of the present disclosure. It should be pointed out that for those of ordinary skill in the art, without departing from the concept of the present disclosure, several modifications and improvements can also be made, which all fall within the protection scope of the present disclosure. Therefore, the protection scope of the present disclosure should be subject to the appended claims.

## Claims

1. A radiation delivery apparatus (10), comprising:
a radiation head (11) configured to deliver a radiation beam and rotatable about a rotation axis; and
a movement mechanism (12) connected to the radiation head (11);
wherein the radiation head (11) is movable along a direction of the rotation axis through the movement mechanism (12), and is movable along a beam direction of the radiation beam through the movement mechanism (12).

2. The radiation delivery apparatus (10) according to claim 1, further comprising:
a first imaging source (13), a first imaging detector (14) corresponding to the first imaging source (13), a second imaging source (15), and a second imaging detector (16) corresponding to the second imaging source (15).

3. The radiation delivery apparatus (10) according to claim 2, wherein an imaging mode of the first imaging source (13) is different from an imaging mode of the second imaging source (15).

4. The radiation delivery apparatus (10) according to claim 3, wherein the imaging mode of the first imaging source (13) comprises a single exposure mode; and
the imaging mode of the second imaging source (15) comprises a continuous exposure mode.

5. The radiation delivery apparatus (10) according to any one of claims 2 to 4, wherein an imaging modality of the first imaging source (13) is different from an imaging modality of the second imaging source (15);
optionally, the imaging modality of the first imaging source (13) comprises computed tomography; and
the imaging modality of the second imaging source (15) comprises digital radiography.

6. The radiation delivery apparatus (10) according to any one of claims 2 to 5, wherein a type of imaging radiation beam of the First imaging source (13) is different from a type of imaging radiation beam of the Second imaging source (15).

7. The radiation delivery apparatus (10) according to any one of claims 2 to 6, wherein a type of the first imaging detector (14) is different from a type of the second imaging detector (16).

8. The radiation delivery apparatus (10) according to any one of claims 2 to 7, wherein a type of the first imaging detector (14) and a type of the second imaging detector (16) comprise a flat panel detector.

9. The radiation delivery apparatus (10) according to any one of claims 2 to 8, wherein a size of the first imaging detector (14) is different from a size of the second imaging detector (16).

10. The radiation delivery apparatus (10) according to any one of claims 1 to 9, wherein the movement mechanism (12) comprises a first movement channel and a second movement channel, the radiation head (11) is disposed in the first movement channel, and the first movement channel is disposed in the second movement channel;
the first movement channel is configured to guide the radiation head (11) to move along a target direction;
the second movement channel is configured to guide the radiation head (11) to move along other directions other than the target direction; and
the target direction comprises the direction of the rotation axis or the beam direction;
optionally, the first movement channel comprises a first guide rail (121), the second movement channel comprises a second guide rail (122), the first guide rail (121) is movably disposed on the second guide rail (122), the radiation head (11) is movably disposed on the first guide rail (121), the second guide rail (122) is configured to guide the first guide rail (121) to move along the direction of the rotation axis, and the first guide rail (121) is configured to guide the radiation head (11) to move along the beam direction.

11. The radiation delivery apparatus (10) according to any one of claims 1 to 9, wherein the movement mechanism (12) comprises a swingable frame (123) and a third guide rail (124), the third guide rail (124) is disposed on the swingable frame (123), the radiation head (11) is movably disposed on the third guide rail (124), the swingable frame (123) is configured to drive the third guide rail (124) to swing along the direction of the rotation axis, and the third guide rail (124) is configured to guide the radiation head (11) to move along the beam direction;
optionally, the radiation delivery apparatus (10) further comprises a radiation detector (17) disposed on the swingable frame (123) and opposite to the radiation head (11).

12. The radiation delivery apparatus (10) according to any one of claims 1 to 11, wherein the radiation head (11) is configured to:
deliver a radiation beam with a first energy when moving to a first position along the beam direction; and
deliver a radiation beam with a second energy different from the first energy when moving to a second position different from the first position along the beam direction;
optionally, the radiation head (11) is configured to deliver the radiation beam focused onto a region of interest on the rotation axis;
a distance between the first position and the region of interest is smaller than a distance between the second position and the region of interest; and
the first energy is smaller than the second energy.

13. The radiation delivery apparatus (10) according to any one of claims 1 to 12, wherein the radiation head (11) comprises a multi-leaf collimator (18); and
the multi-leaf collimator (18) comprises at least two leaf groups, and the at least two leaf groups are configured to be movable independently relative to each other;
optionally, the radiation head (11) is configured to:
deliver a radiation beam to a first target site in a region of interest when at least one of the at least two leaf groups moves to a first position along the beam direction; and
deliver a radiation beam to a second target site different from the first target site in the region of interest when at least one of the at least two leaf groups moves to a second position different from the first position along the beam direction.

14. The radiation delivery apparatus (10) according to claim 13, wherein the radiation head (11) is configured to:
deliver a radiation beam with a first energy when at least one of the at least two leaf groups moves to a third position along the beam direction; and
deliver a radiation beam with a second energy different from the first energy when at least one of the at least two leaf groups moves to a fourth position different from the third position along the beam direction.

15. A non-transitory computer-readable storage medium, on which a computer program is stored, wherein when executed by a processor, the computer program causes a radiation delivery apparatus (10) according to any one of claims 1 to 14 to perform a radiation delivery.
